# EUROPÄISCHE PATENTANMELDUNG

(11) **EP 3 151 025 A2**
(43) Veröffentlichungstag der Anmeldung: **05.04.2017**
(21) Anmeldenummer: 16182574.0
(22) Anmeldetag: 03.08.2016
(51) Int. Cl.: G01R 33/34, G01R 33/341, G01R 33/3415

(54) **ADAPTIVE MR-LOKALSPULE**

(30) Priorität: 29.09.2015 DE 102015218749
(71) Anmelder: Siemens Healthcare GmbH, 91052 Erlangen (DE)
(72) Erfinder: Gruber, Bernhard, 4792 Münzkirchen (AT); Jahns, Karsten, 91054 Buckenhof (DE); Zink, Stephan, 91054 Erlangen (DE)

(57) **Zusammenfassung**

Die Erfindung betrifft eine adaptive MR-Lokalspule, die zumindest eine Leitungsstruktur umfasst. Die zumindest eine Leitungsstruktur ist vorteilhafterweise elektrisch leitfähig, wobei die zumindest eine Leitungsstruktur zumindest teilweise dehnbar und/oder biegbar ist.

## Beschreibung

Die Erfindung betrifft eine adaptive MR-Lokalspule.

In der Medizintechnik stellen bildgebende Verfahren wichtige Hilfsmittel dar. So zeichnet sich etwa in der klinischen Schnittbildgebung Bildgebung mittels Magnetresonanz (MR), auch Magnetresonanztomographie (MRT) genannt, durch hohe und variable Weichteilkontraste aus. Hierbei werden hochfrequente elektromagnetische Magnetresonanzsignale durch elektrisch leitende Schleifen, sogenannten Spulen und/oder Antennen, empfangen. Dabei wird durch das Magnetresonanzsignal eine Spannung in der Spule induziert. Diese Empfangsspulen werden idealerweise möglichst nahe an einem Untersuchungsobjekt, insbesondere einem Patienten, angeordnet. Deshalb werden diese auch als Lokalspulen (engl. local coils) bezeichnet. Die induzierte Spannung wird üblicherweise mittels eines rauscharmen Vorverstärkers verstärkt an die Empfangselektronik weitergeleitet.

Ursprünglich wurden Arrays mit möglichst vielen kleinen Antennen eingeführt, um das Signal-zu-Rauch-Verhältnis (engl. Signal-to-Noise-Ratio, SNR) des empfangenen Magnetresonanzsignals zu verbessern. Das SNR wird meist von den Verlusten in den Antennen, einem schlechten Füllfaktor und möglichen Anwenderfehlern (wie z.B. Überlappungen) negativ beeinflusst.

Inzwischen werden Arrays insbesondere für parallele Bildgebungstechniken genutzt, wie z.B. GeneRalized Autocalibrating Partially Parallel Acquisitions (GRAPPA) und/oder SENSitivity Encoding (SENSE), woraus eine Verkürzung einer Messzeit erzielt werden kann, die für die Untersuchung des Untersuchungsobjekts notwendig ist. Somit besteht ein Bedarf an vielkanaligen Arrays, deren Antennen unterschiedliche Ausrichtungen im Raum relativ zum Sendefeld haben können.

Benutzerfreundlicher Umgang und gutes SNR sind Voraussetzungen für ein optimales Ergebnis und werden mit der steigenden Anzahl von Antennen schwieriger. Heute gibt es verschiedenste Lokalspulentypen, meist für bestimmte anatomische Regionen, wie z.B. Kopf, Knie, Brust, Herz, Prostata, Fuß- Handgelenke und Schulter. Die Anpassung an eine spezifische Patientenform ist bei heutigen Lokalspulen jedoch in der Regel verbesserungsfähig, weil diese oftmals für eine durchschnittliche Patientenform konstruiert werden. Dies führt insbesondere zu reduzierten Füllfaktoren und/oder erhöhten Verkopplungen und damit zu einem schlechteren SNR.

Es ist Aufgabe der vorliegenden Erfindung, eine verbesserte geometrische Anpassungsfähigkeit einer Lokalspule an unterschiedliche Formen eines Untersuchungsobjekts zu ermöglichen.

Diese Aufgabe wird durch die Merkmale der unabhängigen Patentansprüche gelöst. Vorteilhafte Weiterbildungen sind in den Unteransprüchen und der Beschreibung angegeben.

Demnach umfasst die Lokalspule zumindest eine Leitungsstruktur. Die zumindest eine Leitungsstruktur ist vorteilhafterweise elektrisch leitfähig, wobei die zumindest eine Leitungsstruktur zumindest teilweise dehnbar und/oder biegbar ist.

Die zumindest eine Leitungsstruktur kann von einer oder mehreren Antennen umfasst werden. Die eine oder mehreren Antennen können von einem Antennenarray umfasst werden. Die Lokalspule kann mehrere Leitungsstrukturen umfassen, die zumindest teilweise überlappend anordbar sind.

Die elektrische Leitfähigkeit der zumindest einen Leitungsstruktur beträgt bevorzugt mindestens 10³ S/m, besonders bevorzugt mindestens 10⁵ S/m, insbesondere mindestens 10⁷ S/m. Dadurch kann effizient eine Spannung in der einen oder den mehreren Antennen mittels Magnetresonanzsignalen induziert werden. Üblicherweise weist die zumindest eine Leitungsstruktur eine Form einer Schleife (engl. loop) auf, d.h. die Leitungsstruktur kann von einer Spulenschleife umfasst werden. Dadurch kann die zumindest eine Leitungsstruktur als Induktionsschleife und/oder Magnetantenne wirken. Die zumindest eine Leitungsstruktur ist also vorzugsweise zum Senden und Empfangen von Magnetresonanzsignalen ausgebildet.

Bevorzugt ist die zumindest eine Leitungsstruktur in zumindest einer, bevorzugt zumindest zwei, idealerweise in allen drei Raumrichtungen, die optional senkrecht zueinander orientiert sind, dehnbar und/oder biegsam.

Durch die Dehnbarkeit und/oder Biegbarkeit der zumindest einen Leitungsstruktur kann eine hohe geometrische Anpassungsfähigkeit an eine etwaige Form eines Untersuchungsobjekts erreicht werden. Insbesondere kann die zumindest eine Leitungsstruktur ausgebildet sein, reversibel ihre Form zu ändern, d.h. die Form der zumindest einen Leitungsstruktur kann unterschiedliche geometrische Zustände annehmen, die vorzugsweise wiederholt ineinander überführbar sind. Insbesondere sind durch die Dehnbarkeit die Länge und/oder der Umfang der zumindest einen Leitungsstruktur veränderbar. Vorteilhafterweise bleibt dabei die zumindest eine Leitungsstruktur als Ganzes bestehen.

Vorteilhafterweise ist die Lokalspule also so konstruiert, dass sie durch eine Veränderung einer Größe und/oder einer Form der Antennen und/oder durch eine Veränderung der relativen Position der Antennen zueinander an eine Form eines Untersuchungsobjekts anpassbar ist. Dadurch können die eine oder mehrere Antennen sowie die gesamte Lokalspule unabhängig von Form und Größe des Untersuchungsobjekts, insbesondere eines zu untersuchenden Körperteils eines Patienten, so nahe wie möglich an der Oberfläche des Untersuchungsobjekts platziert werden, d.h. durch die geometrische Anpassbarkeit kann der Füllfaktor, also eine Ausfüllung eines von der Lokalspule umschlossenen Volumens durch das Untersuchungsobjekts, optimiert werden. Dies führt vorteilhafterweise zu einer erhöhten Empfindlichkeit und einem gesteigerten SNR.

Die Dehnbarkeit kann insbesondere dadurch erreicht werden, indem die zumindest eine Leitungsstruktur Kohlenstoffnanoröhrchen umfasst. Die Verwendung von Kohlenstoffnanoröhrchen (engl. carbon nanotubes) erlaubt eine Herstellung elastischer elektrischer Leiter, d.h. ein elektrisch leitfähiges Material, das die elektrische Leitfähigkeit der zumindest einen Leitungsstruktur hervorruft, ist vorteilhafterweise selbst dehnbar und/oder elastisch. Insbesondere kann die zumindest eine Leitungsstruktur nach Einwirkung einer Kraft in eine Ursprungsform zurückkehren. Vorteilhafterweise können die Kohlenstoffnanoröhrchen umfassenden Leitungsstrukturen um mindestens 30%, bevorzugt mindestens 60%, besonders bevorzugt mindestens 100% gedehnt werden, ohne mechanisch oder elektrisch beschädigt zu werden.

Ferner wird vorgeschlagen, dass die zumindest eine Leitungsstruktur Einzellitzen und/oder Volldrähte umfasst. Eine Einzellitze besteht üblicherweise aus einem dünnen Einzeldraht, deren Durchmesser vorteilhafterweise mindestens so groß wie die Skin-Tiefe, also die Eindringtiefe aufgrund des Skin-Effekts, ausgelegt wird. Die Skin-Tiefe ist unter anderem abhängig vom angelegten Magnetfeld, welches meist zwischen 1,5 und 7 T liegt. Ein zu großer Durchmesser schränkt die Flexibilität und/oder Biegsamkeit der Einzellitze wiederum ein. Der Durchmesser einer Einzellitze beträgt daher bevorzugt zwischen 3 und 80 µm, besonders bevorzugt zwischen 5 bis 40 µm.

Insbesondere lässt sich aus mehreren Einzellitzen eine Litze herstellen, die bei einem geringen elektrischen Widerstand pro Längeneinheit eine hohe mechanische Flexibilität aufweist. Die Einzellitzen und/oder Volldrähte können insbesondere Kupfer und/oder beschichtete Kupferleiter umfassen.

Bevorzugt weist die zumindest eine Leitungsstruktur zumindest ein textiles Gebilde, insbesondere ein Gewebe und/oder ein Gewirke und/oder Gestricke und/oder ein Geflecht auf, d.h. das zumindest eine textiles Gebilde kann insbesondere gewebt und/oder gewirkt und/oder gestrickt und/oder geflochten und/oder gewunden usw. ausgebildet sein. Unter dem zumindest einem textilen Gebilde kann insbesondere eine ineinander geschlungene Anordnung mehrerer Stränge aus biegsamem Material, insbesondere aus metallischen Drähten und/oder Adern, verstanden werden. Das textile Gebilde kann also beispielsweise ein Drahtgeflecht umfassen.

Das zumindest eine textile Gebilde kann insbesondere räumlich, beispielsweise schlauchförmig, ausgebildet sein. Eine räumliche Ausbildung des textilen Gebildes kann vorsehen, dass das textile Gebilde in einem Querschnitt zu einer Leitungsrichtung eine geschlossene Anordnung umfasst. Die geschlossene Anordnung kann beispielsweise kreisförming oder oval ausgebildet sein. Die Leitungsrichtung kann die Richtung sein, in der ein elektrischer Strom, insbesondere ein elektrischer Nettostrom, induzierbar ist.

Das zumindest eine textile Gebilde kann auch flächenförmig ausgebildet sein. Eine flächenförmige Ausbildung des textilen Gebildes kann vorsehen, dass das textile Gebilde in einem Querschnitt zu der Leitungsrichtung eine offene Anordnung aufweist. Die offene Anordnung kann ein erstes Ende und ein zweites Ende umfassen, wobei das erste Ende und das zweite Ende voneinander beabstandet sind. Die offene Anordnung kann beispielsweise linienförmig ausgebildet sein.

Bevorzugt umfasst das zumindest eine textile Gebilde in einer flächenförmigen Ausbildung eine Fläche. Bevorzugt ist die räumliche Erstreckung des zumindest einen textilen Gebildes in einer flächenförmige Ausbildung in einer ersten Richtung, die insbesondere senkrecht zur Fläche ausgerichtet ist, wesentlich kleiner, bevorzugt vier Mal kleiner, besonders bevorzugt zehn Mal kleiner, als die räumliche Erstreckung in zur ersten Richtung orthogonalen Richtungen. Insbesondere die die Leitungsrichtung orthogonal zur ersten Richtung.

Mittels flächenförmiger Ausbildung können einfache Ausführungsformen mit besonders guten Dehnungseigenschaften realisiert werden.

Für den Fall, dass die Fläche des flächenförmigen textilen Gebildes senkrecht zur Ebene einer Spulenschleife orientiert ist, weist das flächenförmige textile Gebilde eine besonders hohe Flexibilität auf. Denn im Gegensatz zu einer räumlichen, insbesondere schlauchförmigen Ausbildung, weist ein flächenförmiges textiles Gebilde senkrecht zu seiner Nulllinie, die auch als neutrale Faser bezeichnet werden kann und üblicherweise parallel zur ersten Richtung verläuft, eine geringe Erstreckung auf, so dass hier bei Änderung der Form der Leitungsstruktur geringere Stauchungen und/oder Dehnungen bzw. geringere Biegeverkürzungen und/oder Biegeverlängerungen auftreten. Dies erhöht die Biegsamkeit und verringert die Knickgefahr.

Die Fläche eines flächenförmigen textilen Gebildes kann aber natürlich auch parallel zur Ebene der Spulenschleife orientiert sein und/oder in einem beliebigen Winkel zur Ebene der Spulenschleife geneigt sein. Insbesondere kann die Orientierung der Fläche des flächenförmigen textilen Gebildes zur Ebene der Spulenschleife abschnittsweise variieren.

Vorteilhafterweise weist das zumindest eine textile Gebilde also eine hohe Dehnbarkeit und/oder Biegsamkeit auf, wodurch eine flexible geometrische Anpassung der Lokalspule möglich wird.

Ferner umfasst die Lokalspule mehrere Leitungsstrukturen, die eine gleiche Vorzugsrichtung aufweisen. Insbesondere können die mehreren Leitungsstrukturen parallel zueinander anordbar sein. Durch eine gemeinsame Vorzugsrichtung kann eine systematische Abdeckung des Untersuchungsobjekts erreicht werden.

Ferner können somit unkompliziert die mehreren Leitungsstrukturen regelmäßig angeordnet werden. Zudem können die mehreren Leitungsstrukturen unterschiedlich beabstandet sein, wodurch eine verbesserte Anpassbarkeit der Lokalspule ermöglicht wird.

Bevorzugt umfasst die Lokalspule mehrere Leitungsstrukturen, die gekreuzt, insbesondere gitterförmig, angeordnet sind. Dadurch lassen sich beispielsweise geometrisch flexible Matten und/oder Gewebe erzeugen, die gut an unterschiedliche Formen anpassbar sind.

Eine Ausführungsform sieht vor, dass die Lokalspule zumindest eine, insbesondere elektrisch isolierende, Trägerstruktur umfasst.

Das elektrisch isolierende Trägermaterial weist üblicherweise eine elektrische Leitfähigkeit von weniger als 1 S/m auf. Die Trägerstruktur kann die zumindest eine Leitungsstruktur stützen, d.h. die Trägerstruktur kann als ein Gerüst für die zumindest eine Leitungsstruktur dienen. Mittels der zumindest einen Trägerstruktur können die Gestaltungsmöglichkeiten und/oder Anpassungsmöglichkeiten der Lokalspulenform erhöht werden.

Vorteilhafterweise ist die zumindest eine Trägerstruktur kompatibel für MR-Bildgebung, insbesondere besteht die Trägerstruktur aus einem Material, das MR-stumm ist und keine MR-Signale absorbiert.

Bevorzugt ist die zumindest eine Trägerstruktur zumindest teilweise dehnbar und/oder biegsam.

Durch eine Kombination von einer zumindest teilweise dehnbaren und/oder biegsamen Trägerstruktur mit einer Leitungsstruktur kann eine dehnbare Leiterschleife realisiert werden.

Um eine dehnbare Gesamtanordnung zu ermöglichen, kann es ausreichend sein, dass nur ein Teil der Trägerstruktur dehnbar und/oder biegsam ist, d.h. die Trägerstruktur kann auch starre Elemente umfassen.

Bevorzugt ist die zumindest eine Leitungsstruktur dabei an der zumindest einen Trägerstruktur angeordnet, insbesondere umwebt und/oder umwunden und/oder umstrickt usw. Die die zumindest eine Leitungsstruktur und die zumindest einen Trägerstruktur können also zusammen ein textiles Gebilde ausweisen. Diese Ausführungsformen ermöglichen eine hohe mechanische Anpassungsfähigkeit.

Eine Ausführungsform sieht vor, dass die Lokalspule mehrere Trägerstrukturen umfasst, die parallel zueinander angeordnet sind. Eine derartige Struktur eignet sich insbesondere gut für eine geometrische Anpassung an längliche Untersuchungsobjekte, wie beispielsweise Arme und/oder Beine.

Ferner wird vorgeschlagen, dass die zumindest eine Leitungsstruktur einen zickzackförmigen und/oder mäanderförmigen Verlauf aufweist. Leitungsstrukturen mit derartigen Verläufen lassen sich besonders einfach strecken und/oder stauchen und damit besonders einfach an verschiedene Patientenanatomien anpassen.

Eine Ausführungsform sieht vor, dass die zumindest eine Leitungsstruktur auf einem Zylinder, insbesondere Kreiszylinder, anordbar ist. Bevorzugt umschließt der Kreiszylinder ein Volumen, in dem ein Untersuchungsobjekt, insbesondere ein Körperteil eines Patienten wie beispielsweise ein Bein und/oder ein Arm, aufnehmbar ist.

Bevorzugt ist die zumindest eine Leitungsstruktur zumindest abschnittsweise federnd ausgebildet. Insbesondere weist die zumindest eine Leitungsstruktur zumindest eine Feder auf. Bevorzugt umfasst die zumindest eine Trägerstruktur bis zu sechs Federn, besonders bevorzugt bis zu vier Federn, insbesondere bis zu zwei Federn.

Eine federnde Anordnung stellt durch die Dehnbarkeit von Federn sowie deren Rückstellkraft eine gute Möglichkeit zur anatomischen Anpassung dar. Mit einer Teilfeder-Anordnung, also einer Anordnung, die abschnittsweise federnde und starre Bereiche umfasst, lässt sich eine robuste und trotzdem insgesamt flexible und dehnbare Leitungsstruktur erzeugen. Die elektrische Leitfähigkeit der Leitungsstruktur lässt sich durch das Material, aus dem die Federn bestehen, beeinflussen. Damit lässt sich auch eine Güte einer etwaigen Antenne, die die Leitungsstruktur umfasst, beeinflussen.

Die Federn selbst können in unterschiedlichen Formen ausgeführt sein, wie z.B. als Schraubenfeder und/oder als konische Feder und/oder als Teleskopfeder und/oder als flache Feder. Eine Schraubenfeder weist üblicherweise einen helikalen Verlauf auf, d.h. der Verlauf beschreibt eine Kurve, die sich um den Mantel eines Zylinders windet. Beispielsweise kann dabei ein Runddraht und/oder ein Flachdraht verwendet werden. Eine Kegelfeder weist üblicherweise einen konischen Verlauf auf, d.h. der Verlauf beschreibt eine Kurve, die sich um den Mantel eines Kegels windet. Eine flache Feder weist üblicherweise ein federndes Gebilde auf, welches in einer Ebene verläuft. Dabei kann das federnde Gebilde einen mäandrierenden Draht umfassen. Derartige Federtypen eigenen sich besonders gut, da sich in der Regel eine gute Verfügbarkeit und eine hohe Dehnbarkeit aufweisen.

Ferner kann die Lokalspule zumindest eine Schutzummantelung umfassen, die die zumindest eine Leitungsstruktur herum angeordnet ist. Die Schutzummantelung weist bevorzugt einen Durchmesser bis zu 10 mm, insbesondere bis zu 5 mm auf.

Ferner umfasst die Lokalspule vorzugsweise zumindest eine Anpassschaltung. Durch Dehnung der zumindest einen Trägerstruktur kann sich eine veränderte Impedanz und/oder Eigenfrequenz einstellen. Die Impedanz an einem Eingang eines etwaigen Vorverstärkers kann sich auch bedingt durch eine unterschiedliche Beladung (engl. loading) der zumindest einen Leitungsstruktur, Wechselwirkungen zwischen der zumindest einen Leitungsstruktur und einem Untersuchungsobjekt sowie einer Kopplung zwischen mehreren Leitungsstrukturen ändern. Diese Impedanzveränderungen und/oder Frequenzverschiebungen können vorteilhafterweise durch die zumindest eine Anpassschaltung kompensiert werden. Dadurch kann das Rauschverhalten, insbesondere eine Rauschzahl des etwaigen Vorverstärkers, die stark von der Impedanz am Eingang und einer Messfrequenz abhängt, verbessert und ein höheres SNR erzielt werden.

Ferner wird eine Magnetresonanzvorrichtung mit einer erfindungsgemäßen Lokalspule vorgeschlagen. Die Vorteile der Magnetresonanzvorrichtung entsprechen im Wesentlichen den Vorteilen der Lokalspule, die vorab im Detail beschrieben worden sind.

Weitere Vorteile, Merkmale und Einzelheiten der Erfindung ergeben sich aus den im Folgenden beschriebenen Ausführungsbeispielen sowie anhand der Zeichnungen. Einander entsprechende Teile sind in allen Figuren mit den gleichen Bezugszeichen versehen.

Es zeigen:
- Fig. 1: eine schematische Darstellung einer erfindungsgemäßen Magnetresonanzvorrichtung,
- Fig. 2: eine schematische Darstellung einer Lokalspule mit mehreren Leitungsstrukturen, die gekreuzt angeordnet sind,
- Fig. 3: eine schematische Darstellung einer Lokalspule mit mehreren Leitungsstrukturen, die auf einem Kreiszylinder angeordnet sind,
- Fig. 4: eine schematische Darstellung eines Ausschnitts einer Leitungsstruktur, die ein textiles Gebilde aufweist,
- Fig. 5: eine schematische Darstellung einer schleifenförmigen Leitungsstruktur, die ein textiles Gebilde aufweist,
- Fig. 6: eine schematische Darstellung eines Querschnitts eines flächenförmigen textilen Gebildes,
- Fig. 7: eine schematische Darstellung eines Ausschnitts einer Leitungsstruktur, die ein gestricktes Gebilde aufweist,
- Fig. 8: eine schematische Darstellung eines Ausschnitts einer Leitungsstruktur, die ein gewobenes Gebilde aufweist,
- Fig. 9: eine schematische Darstellung eines Ausschnitts einer Leitungsstruktur, die ein gewickeltes Gebilde aufweist,
- Fig. 10: eine schematische Darstellung einer manschettenförmigen Lokalspule,
- Fig. 11: eine schematische Darstellung einer mattenförmigen Lokalspule,
- Fig. 12: eine schematische Darstellung einer Leitungsstruktur, die durchgehend federnd ausgebildet ist,
- Fig. 13: eine schematische Darstellung einer Leitungsstruktur, die abschnittsweise federnd ausgebildet ist,
- Fig. 14: eine schematische Darstellung eines Arrays von Leitungsstrukturen, die abschnittsweise federnd ausgebildet sind,
- Fig. 15: eine schematische Darstellung eines Ausschnitts einer Leitungsstruktur mir einer Schutzummantelung.

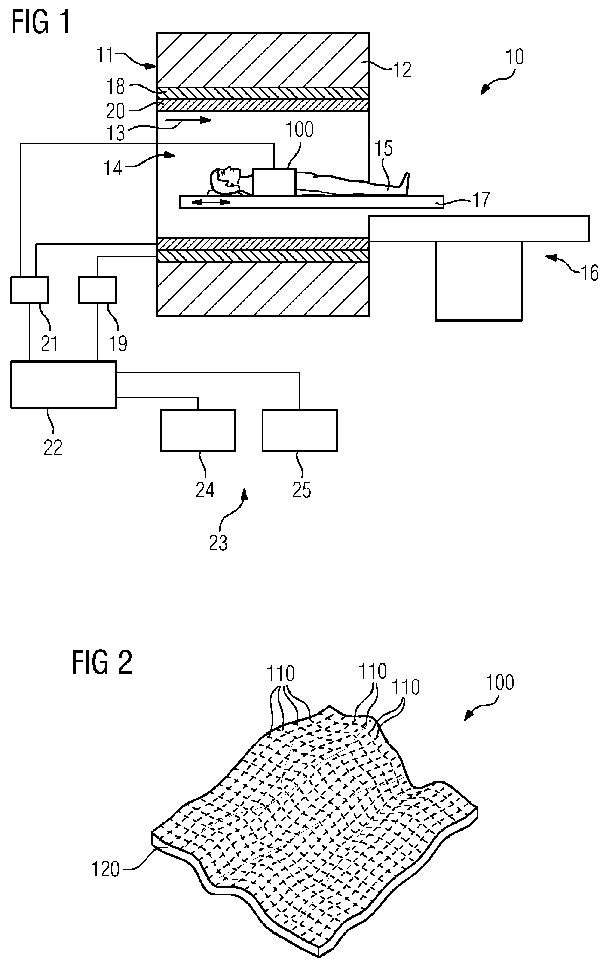

In Fig. 1 ist eine Magnetresonanzvorrichtung 10 schematisch dargestellt. Die Magnetresonanzvorrichtung 10 umfasst eine Magneteinheit 11, die einen supraleitenden Hauptmagneten 12 zu einem Erzeugen eines starken und insbesondere zeitlich konstanten Hauptmagnetfelds 13 aufweist. Zudem umfasst die Magnetresonanzvorrichtung 10 einen Patientenaufnahmebereich 14 zu einer Aufnahme eines Patienten 15. Der Patientenaufnahmebereich 14 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 14 jederzeit denkbar. Der Patient 15 kann mittels einer Patientenlagerungsvorrichtung 16 der Magnetresonanzvorrichtung 10 in den Patientenaufnahmebereich 14 geschoben werden. Die Patientenlagerungsvorrichtung 16 weist hierzu einen innerhalb des Patientenaufnahmebereichs 14 bewegbar ausgestalteten Patiententisch 17 auf.

Die Magneteinheit 11 weist weiterhin eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der Magnetresonanzvorrichtung 10 gesteuert. Die Magneteinheit 11 umfasst weiterhin eine Hochfrequenzantenneneinheit 20, welche im vorliegenden Ausführungsbeispiel als fest in die Magnetresonanzvorrichtung 10 integrierte Körperspule ausgebildet ist. Die Hochfrequenzantenneneinheit 20 ist zu einer Anregung von Atomkernen, die sich in dem von dem Hauptmagneten 12 erzeugten Hauptmagnetfeld 13 einstellt, ausgelegt. Die Hochfrequenzantenneneinheit 20 wird von einer Hochfrequenzantennensteuereinheit 21 der Magnetresonanzvorrichtung 10 gesteuert und strahlt hochfrequente Magnetresonanzsequenzen in einen Untersuchungsraum ein, der im Wesentlichen von einem Patientenaufnahmebereich 14 der Magnetresonanzvorrichtung 10 gebildet ist. Die Hochfrequenzantenneneinheit 20 ist weiterhin zum Empfang von Magnetresonanzsignalen ausgebildet.

Zu einer Steuerung des Hauptmagneten 12, der Gradientensteuereinheit 19 und zur Steuerung der Hochfrequenzantennensteuereinheit 21 weist die Magnetresonanzvorrichtung 10 eine Systemsteuereinheit 22 auf. Die Systemsteuereinheit 22 steuert zentral die Magnetresonanzvorrichtung 10, wie beispielsweise das Durchführen einer vorbestimmten bildgebenden Gradientenechosequenz. Zudem umfasst die Systemsteuereinheit 22 eine nicht näher dargestellte Auswerteeinheit zu einer Auswertung von medizinischen Bilddaten, die während der Magnetresonanzuntersuchung erfasst werden. Des Weiteren umfasst die Magnetresonanzvorrichtung 10 eine Benutzerschnittstelle 23, die mit der Systemsteuereinheit 22 verbunden ist. Steuerinformationen wie beispielsweise Bildgebungsparameter, sowie rekonstruierte Magnetresonanzbilder können auf einer Anzeigeeinheit 24, beispielsweise auf zumindest einem Monitor, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

Ferner umfasst die Magnetresonanzvorrichtung 10 eine Lokalspule 100, die in diesem Beispiel im Brustbereich des Patienten 15 angeordnet ist. Die Lokalspule 100 umfasst zumindest eine elektrisch leitfähige Leitungsstruktur, wobei die zumindest eine Leitungsstruktur zumindest teilweise dehnbar und/oder biegbar ist. Die Lokalspule 100 ist ebenso wie die Hochfrequenzantenneneinheit 20 zu einer Anregung von Atomkernen und zum Empfang von Magnetresonanzsignalen ausgelegt und wird von der Hochfrequenzantennensteuereinheit 21 gesteuert.

Die dargestellte Magnetresonanzvorrichtung 10 im vorliegenden Ausführungsbeispiel kann selbstverständlich weitere Komponenten umfassen, die Magnetresonanzvorrichtungen gewöhnlich aufweisen. Eine allgemeine Funktionsweise einer Magnetresonanzvorrichtung 10 ist zudem dem Fachmann bekannt, so dass auf eine detaillierte Beschreibung der allgemeinen Komponenten verzichtet wird.

Fig. 2 zeigt ein Ausführungsbeispiel, in welchem die Lokalspule 100 mehrere Leitungsstrukturen 110 und eine Trägerstruktur 120 umfasst, wobei zur Erhöhung der Übersichtlichkeit die Leitungsstrukturen 110 nur teilweise mit eigenen Bezugszeichen versehen sind. Die Leitungsstrukturen 110 sind als geometrisch anpassbares Gitternetz ausgebildet, d.h. die Leitungsstrukturen 110 sind gekreuzt angeordnet. Das Gitternetz kann sich gut an eine Anatomie des Patienten 15 anschmiegen. Dazu ist das Material, aus dem die Leitungsstrukturen 110 bestehen, vorteilhafterweise selbst dehnbar und/oder elastisch. Elastische Materialien, die gleichzeitig eine hohe elektrische Leitfähigkeit aufweisen, umfassen beispielsweise Kohlenstoffnanoröhrchen. Das Gitternetz kann beispielsweise geflochtene und/oder gewobene elektrische Leiter umfassen. Das Gitternetz wird von einer Trägerstruktur 120 gesäumt, die der Anordnung Stabilität verleiht. Um die Anpassungsfähigkeit der Gesamtanordnung nicht zu behindern, ist auch die Trägerstruktur 120 zumindest teilweise dehnbar und/oder biegsam ausgebildet.

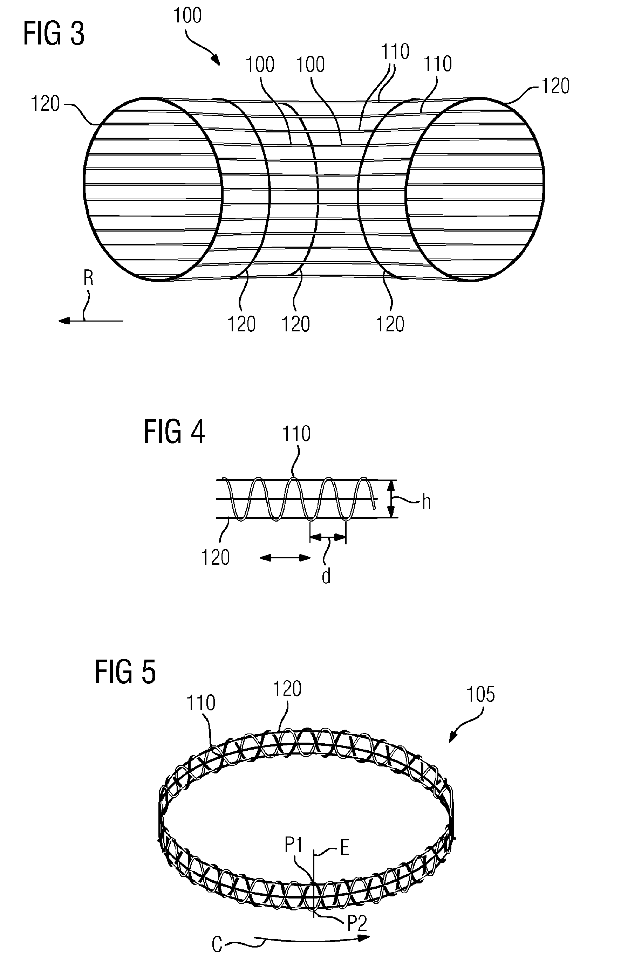

Fig. 3 zeigt ein weiteres Ausführungsbeispiel einer Lokalspule 100 mit einer mehreren Leitungsstrukturen 110, die eine gleiche Vorzugsrichtung R aufweisen und auf einem Zylinder anordbar sind. Innerhalb dieses Zylinders kann ein Untersuchungsobjekt, bevorzugt ein längliches Untersuchungsobjekt wie beispielsweise ein Arm oder ein Bein, positioniert werden. Ferner weisen die mehreren Leitungsstrukturen 110 eine gleiche Vorzugsrichtung R auf.

Darüber hinaus weist die Lokalspule 100 Trägerstrukturen 120 auf, die um die Leitungsstrukturen 110 herum verlaufen. Die Trägerstrukturen 120 sind beispielsweise parallel zueinander angeordnet. Die Trägerstrukturen 120 sind vorteilhafterweise zusammenziehbar, d.h. sie können als Gurte wirken. Dadurch können die Leitungsstrukturen 110 eng an das zu untersuchende Objekt angeordnet werden, so dass sich ein hoher Füllfaktor erzielen lässt.

Zur Realisierung von dehnbaren und/oder biegsamen Leitungsstrukturen eignen sich auch textile Gebilde, wie sie beispielhaft in den Fig. 4 bis 9 dargestellt sind. Die Doppelpfeile illustrieren dabei die mögliche Dehnungsrichtung der der textilen Gebilde. Die textilen Gebilde können elektrische Leiter, wie beispielsweise reine Kupferleiter, beschichtete Kupferleiter oder Leiter aus anderen Metallen umfassen. Zudem können die Leitungsstrukturen Einzellitzen und/oder Volldrähte aufweisen. Die Flexibilität, Dehnbarkeit und/oder Anpassbarkeit wird durch die textile Anordnung der Leiter erzeugt.

Fig. 4 zeigt ein Beispiel eines textilen Gebildes, bei dem eine Leitungsstruktur 110 an einer Trägerstruktur 120 angeordnet ist. Dabei ist die Leitungsstruktur 110 in die Trägerstruktur 120 eingewoben und weist einen zickzackförmigen und/oder mäanderförmigen Verlauf auf.

Die Form des textilen Gebildet ist bevorzugt so ausgebildet, dass ein Abstand d zwischen zwei sich wiederholenden Abschnitten zwischen 4 bis 6 mm beträgt und eine Höhe d des sich wiederholenden Abschnitts zwischen 5 und 7 mm beträgt.

Die Trägerstrukturen 120 sind vorteilhafterweise dehnbar und/oder elastisch ausgebildet. Dazu umfassen die Trägerstrukturen 120 vorzugsweise dehnbare und/oder elastische Materialien wie z.B. Gummi. Die Trägerstrukturen 120 können also beispielsweise Gummibänder umfassen. Die Trägerstrukturen 120 brauchen dabei selbst nicht leitfähig zu sein, da die Leitfähigkeit der Gesamtanordnung durch die Leitungsstrukturen 110 erzielt wird.

Ein textiles Gebildes, wie es beispielhaft in Fig. 4 illustriert wird, kann in Form einer Schleife angeordnet sein, wie sie beispielhaft in Fig. 5 dargestellt wird. Die hier dargestellte Anordnung 105 umfasst eine Leitungsstruktur 110. Diese Leitungsstruktur 110 weist ein textiles Gebilde auf, das zwei Leiter umfasst, die an einer Trägerstruktur 120 angeordnet sind. Die Schleife kann dabei unterschiedliche Ausdehnungszustände annehmen, d.h. die von der Schleife umschlossene Fläche kann verändert werden. Dadurch ist eine gute Anpassungsfähigkeit der Schleife an unterschiedliche Patientenformen gegeben.

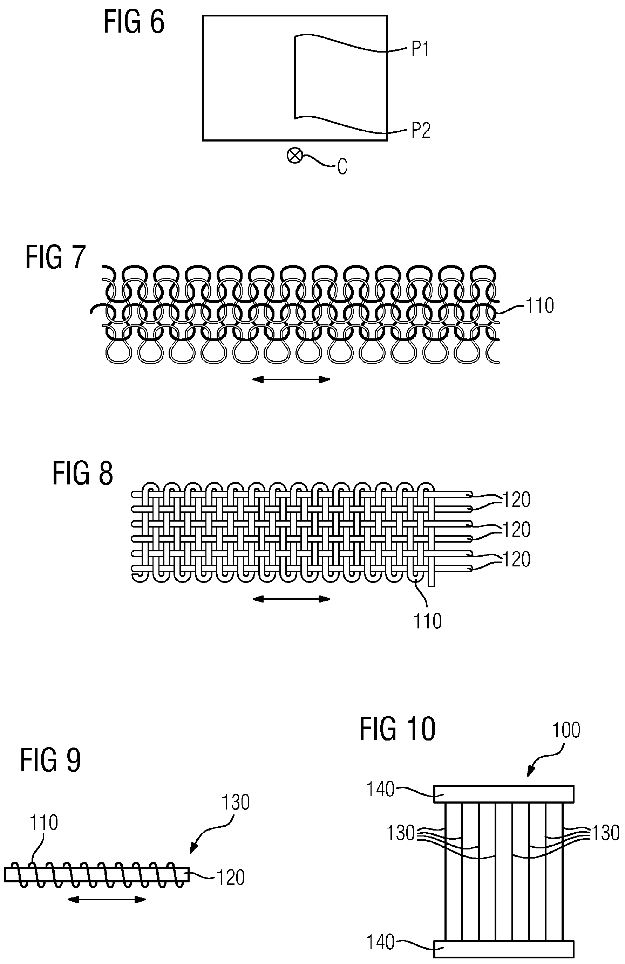

Die in Fig. 5 dargestellte Leitungsstruktur weist ein textiles Gebilde auf, das flächenförmig ausgebildet ist, d.h. das textile Gebilde weist in einem Querschnitt, der stark schematisiert in Fig. 6 dargestellt ist, zu einer Leitungsrichtung C eine offene Anordnung auf. Der Querschnitt liegt dabei beispielsweise in der Ebene E. Die Leitungsrichtung C ist hier eine Richtung, in welcher ein elektrischer Strom fließen kann, der insbesondere durch Magnetresonanzsignale induzierbar ist. Die offene Anordnung umfasst ein erstes Ende P1 und ein zweites Ende P2, wobei das erste Ende P1 und das zweite Ende P2 voneinander beabstandet sind. Die offene Anordnung ist hier linienförmig ausgebildet.

Die Fläche des textilen Gebildes ist hier im Wesentlichen senkrecht zur Ebene der Spulenschleife orientiert. Die Ebene der Spulenschleife ist üblicherweise die Ebene, in der die Leitungsrichtung C liegt. Das bedeutet, dass die Verbindungslinie zwischen den Punkten P1 und P2 im Wesentlichen senkrecht zur Ebene der Spulenschleife und zur Leitungsrichtung C orientiert ist. In dieser Ausbildung weist das flächenförmige textile Gebilde eine besonders hohe Flexibilität auf, da es senkrecht zu seiner Nulllinie eine geringe Erstreckung aufweist, so dass hier bei Änderung der Form der Leitungsstruktur 110 geringere Stauchungen und/oder Dehnungen bzw. geringere Biegeverkürzungen und/oder Biegeverlängerungen auftreten.

Die Fläche eines flächenförmigen textilen Gebildes kann aber natürlich auch parallel zur Ebene der Spulenschleife orientiert sein und/oder in einem beliebigen Winkel zur Ebene der Spulenschleife geneigt sein. Insbesondere kann die Orientierung der Fläche des flächenförmigen textilen Gebildes zur Ebene der Spulenschleife abschnittsweise variieren.

Beispiele für weitere Typen textiler Gebilde sind in den Fig. 7 und 8 dargestellt. Während in Fig. 7 eine gestrickte Leitungsstruktur dargestellt ist, zeigt Fig. 8 eine gewebte Leitungsstruktur. Fig. 8 zeigt ein Beispiel, in dem, ähnlich wie im in Fig. 4 dargestellten Beispiel, eine Trägerstruktur 120 von einer Leitungsstruktur 110 umwoben ist.

Dagegen ist in einem weiteren Beispiel, das in Fig. 9 gezeigt wird, eine Trägerstruktur 120, wie z.B. ein Gummiband oder eine anderes dehnbares Band, von einer Leitungsstruktur 110 umwickelt. Zusammen bilden sie eine elektrisch leitfähige und dabei gleichzeitig dehnbare und/oder elastische Schnüre 130, die beispielsweise zur Realisierung einer Lokalspule 100 in Form einer adaptiven Manschette eingesetzt werden kann, wie sie in Fig. 10 dargestellt wird. Neben den Schnüren 130, die hier parallel zueinander angeordnet sind, umfasst die Lokalspule Befestigungselemente 140, mit der die Lokalspule an einen Patienten 15 abgebracht werden kann.

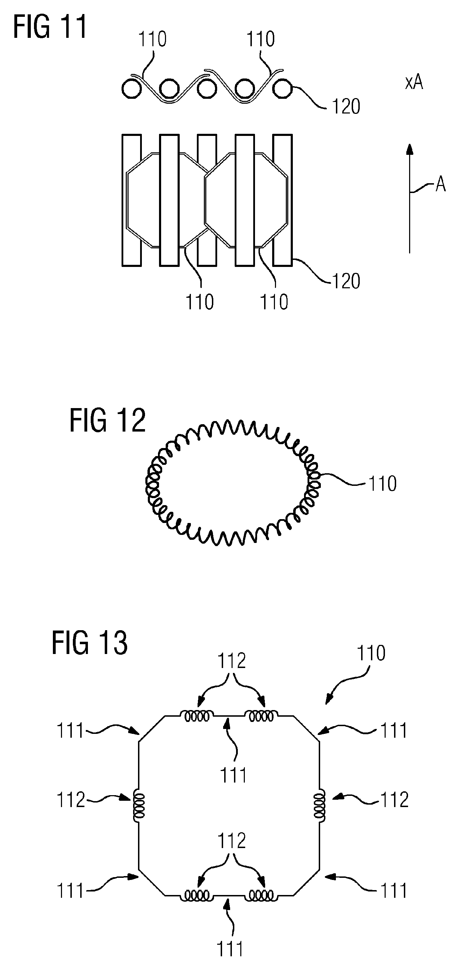

Ein weiteres Beispiel einer adaptiven Lokalspulenkonstruktion wird in Fig. 11 gezeigt. Die Anordnung umfasst hier zwei schleifenförmige Leitungsstrukturen 110, die um stabförmige Trägerstrukturen 120 angeordnet sind, beispielsweise indem ein Trägermaterial mit einem Flachspulenelement 110 um runde Stangen 120 gewickelt wird. Durch diese Anordnungsweise kann die Gesamtanordnung zumindest um die Achse A reversibel gekrümmt werden.

Ferner eignen sich zur Realisierung von dehnbaren und/oder biegsamen Leitungsstrukturen auch Leitungsstruktur, die zumindest abschnittsweise federnd ausgebildet sind. Dies wird anhand der Ausführungsbeispiele illustriert, die in den Fig. 12 bis 14 dargestellt sind.

Eine Federkonstruktion stellt durch die Dehnbarkeit von Federn, insbesondere in Verbindung mit ihrer Rückstellkraft, eine gute Möglichkeit zur geometrischen Anpassung dar. So besteht das in Fig. 12 gezeigte Beispiel eine durchgehend gefederte Schleife als Leitungsstruktur 110 dar, deren Form und Größe anpassbar ist.

In Fig. 13 ist dagegen eine Variante dargestellt mit einer Teil-Feder-Konstruktion, d.h. die Leitungsstruktur 110 umfasst hier sechs starre Bereiche 111 und sechs gefederte Bereiche 112. Selbstverständlich kann eine erfindungsgemäße Leitungsstruktur auch mehr oder weniger starre und/oder gefederte Bereiche aufweisen. Die gefederten Bereiche weisen hier Federn auf, die als Schraubenfedern ausgebildet sind. Durch die gefederten Bereiche ist auch die Gesamtanordnung flexibel. Die elektrische Leitfähigkeit und damit auch indirekt die Güte eines Spulenelements, das die Leitungsstruktur 110 umfassen kann, lässt sich durch das Material beeinflussen, aus dem die Feder besteht.

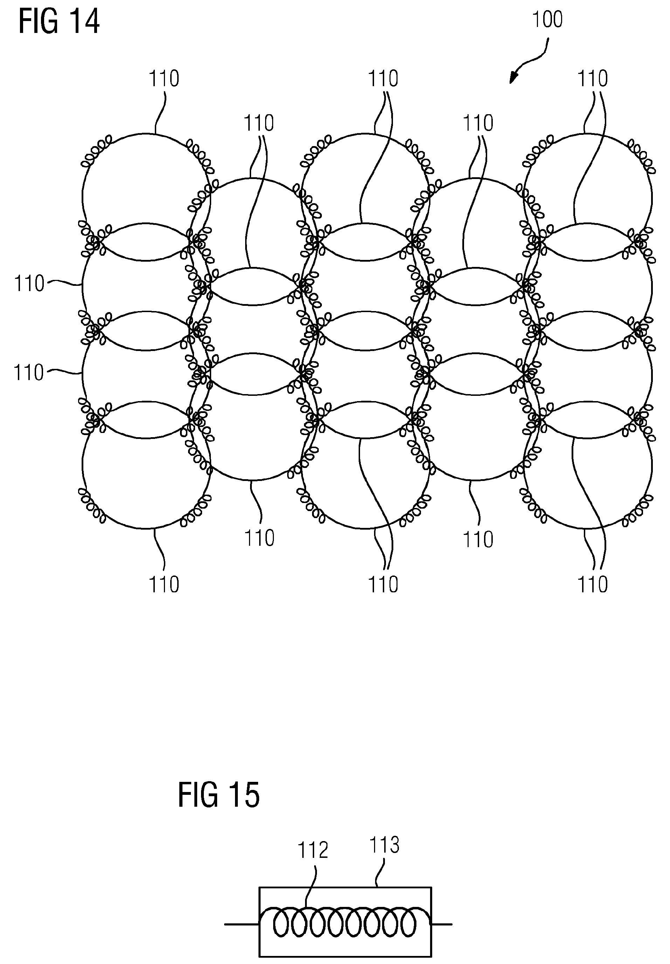

In Fig. 14 ist als Lokalspule 100 ein Array mit achtzehn Leitungsstrukturen 110 dargestellt, d.h. jede der Leitungsstrukturen 110 entspricht einem Spulenelement. Selbstverständlich kann ein Array auch mehr oder weniger Leitungsstrukturen umfassen. Jede der Leitungsstrukturen 110 umfasst vier starre Bereiche und vier gefederte Bereiche. Die starren Bereiche werden beispielsweise durch starre Kupferleiter gebildet. Die gefederten Bereiche können beispielsweise ein Kupfergeflecht und/oder anderes flexibles leitfähiges Material ausweisen. Die Leitungsstrukturen 110 weisen bevorzugt einen Durchmesser von 6 bis 18 cm auf. Vorteilhafterweise sind die Leitungsstrukturen 110 in jeweils starren Bereichen miteinander verbunden.

Die federnden Abschnitte der Leitungsstrukturen können beispielsweise Schraubenfedern und/oder konische Federn und/oder Teleskopfedern und/oder flache Federn umfassen. Ferner ist es möglich, dass die federnden Abschnitte textile Gebilde umfassen, wie sie beispielhaft in den Fig. 4 bis 9 dargestellt sind, da auch textile Gebilde federnde Eigenschaften aufweisen können.

In Fig. 15 ist ferner eine Feder 112 mit einer Schutzummantelung 113 dargestellt, die die Feder 112 vor Beschädigung schützt.

Es wird abschließend darauf hingewiesen, dass es sich bei den vorhergehend detailliert beschriebenen Erzeugnissen lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können.

Zusammenfassend lässt sich festhalten, dass durch die vorliegende Erfindung eine, insbesondere in drei Raumrichtungen, flexible Lokalspule bereitgestellt werden kann. Dies wird erreicht, indem zumindest eine Leitungsstruktur zumindest teilweise dehnbar und/oder biegbar ausgebildet ist.

Ein Einzelspulenelement, das mit Hilfe einer Leitungsstruktur gebildet werden kann, kann also nicht nur flexibel ausgebildet sein, sondern zudem dehnbar, d.h. die von einem Einzelspulenelement umschlossene Fläche kann in einem gewissen Bereich verkleinert und vergrößert werden. Diese Dehnbarkeit kann dazu genutzt werden, ein etwaiges Lokalspulen-Array, das mehrere Einzelspulenelemente umfasst, an verschiedene, insbesondere menschliche, Anatomien anzupassen.

Diese Anpassbarkeit kann beispielsweise durch elektrisch leitfähige Federn, durch Drahtgeflechte, leitfähig beschichtete dehnbare Basismaterialien und/oder sonstige dehnbare elektrisch leitfähige Materialien erzeugt werden.

Durch Anpassbarkeit können Lokalspulen erzeugt werden, die sich für die Untersuchung verschiedener Körperbereiche, wie z.B. Abdomen, Hüfte, Knie, Sprunggelenk, Schulter, etc., einsetzen lassen. Somit werden weniger spezifische Lokalspulen benötigt. Ferner wird der Patientenkomfort, das Handling, der Arbeitsablauf und die Gebrauchstauglichkeit von Lokalspulen vereinfacht und verbessert.

## Patentansprüche

1. Lokalspule mit zumindest einer Leitungsstruktur,
wobei die zumindest eine Leitungsstruktur elektrisch leitfähig ist,
wobei die zumindest eine Leitungsstruktur zumindest teilweise dehnbar und/oder biegbar ist.

2. Lokalspule nach Anspruch 1,
wobei die zumindest eine Leitungsstruktur Kohlenstoffnanoröhrchen umfasst.

3. Lokalspule nach einem der vorangehenden Ansprüche,
wobei die zumindest eine Leitungsstruktur Einzellitzen und/oder Volldrähte umfasst.

4. Lokalspule nach einem der vorangehenden Ansprüche,
wobei die zumindest eine Leitungsstruktur zumindest ein textiles Gebilde aufweist.

5. Lokalspule nach Anspruch 4,
wobei das zumindest eine textiles Gebilde gewebt und/oder gewirkt und/oder gestrickt und/oder geflochten und/oder gewunden ausgebildet ist.

6. Lokalspule nach einem der Ansprüche 4 oder 5,
wobei das zumindest eine textile Gebilde räumlich ausgebildet ist.

7. Lokalspule nach einem der Ansprüche 4 bis 6,
wobei das zumindest eine textile Gebilde flächenförmig ausgebildet ist.

8. Lokalspule nach einem der vorangehenden Ansprüche,
wobei die Lokalspule mehrere Leitungsstrukturen umfasst, die eine gleiche Vorzugsrichtung aufweisen.

9. Lokalspule nach einem der vorangehenden Ansprüche,
wobei die Lokalspule mehrere Leitungsstrukturen umfasst, die gekreuzt angeordnet sind.

10. Lokalspule nach einem der vorangehenden Ansprüche,
wobei die Lokalspule zumindest eine Trägerstruktur umfasst.

11. Lokalspule nach Anspruch 10,
wobei die zumindest eine Trägerstruktur zumindest teilweise dehnbar und/oder biegsam ist.

12. Lokalspule nach einem der Ansprüche 10 oder 11,
wobei die zumindest eine Leitungsstruktur an der zumindest einen Trägerstruktur angeordnet ist.

13. Lokalspule nach einem der Ansprüche 10 bis 12,
wobei die Lokalspule mehrere Trägerstrukturen umfasst, die parallel zueinander angeordnet sind.

14. Lokalspule nach einem der vorangehenden Ansprüche, wobei die zumindest eine Leitungsstruktur einen zickzackförmigen und/oder mäanderförmigen Verlauf aufweist.

15. Lokalspule nach einem der vorangehenden Ansprüche,
wobei die zumindest eine Leitungsstruktur auf einem Zylinder anordbar ist.

16. Lokalspule nach einem der vorangehenden Ansprüche,
wobei die zumindest eine Leitungsstruktur zumindest abschnittsweise federnd ausgebildet ist.

17. Lokalspule nach Anspruch 16,
wobei die zumindest abschnittsweise federnd ausgebildete zumindest eine Leitungsstruktur zumindest eine Feder umfasst.

18. Lokalspule nach Anspruch 17,
wobei die zumindest eine Feder zumindest eine Schraubenfeder und/oder konische Feder und/oder Teleskopfeder und/oder flache Feder umfasst.

19. Lokalspule nach einem der vorangehenden Ansprüche,
wobei die Lokalspule zumindest eine Schutzummantelung umfasst, die die zumindest eine Leitungsstruktur herum angeordnet ist.

20. Lokalspule nach einem der vorangehenden Ansprüche,
wobei die Lokalspule zumindest eine Anpassungsschaltung umfasst.

21. Magnetresonanzvorrichtung mit einer Lokalspule nach einem der Ansprüche 1 bis 20.
